# EUROPÄISCHE PATENTSCHRIFT

(11) **EP 0 837 853 B1**
(45) Veröffentlichungstag und Bekanntmachung des Hinweises auf die Patenterteilung: **20.11.2002**
(21) Anmeldenummer: 96922004.5
(22) Anmeldetag: 17.06.1996
(51) Int. Cl.: C07D 239/54, C07D 405/12, A01N 43/54

(54) **SUBSTITUIERTE CYANOPHENYLURACILE**
SUBSTITUTED CYANOPHENYL URACILS
CYANOPHENYLURACILES SUBSTITUES

(30) Priorität: 29.06.1995 DE 19523650; 19.12.1995 DE 19547475
(43) Veröffentlichungstag der Anmeldung: 29.04.1998
(60) Teilanmeldung: 02009276.3
(73) Patentinhaber: BAYER AG, 51368 Leverkusen (DE); NIHON BAYER AGROCHEM K.K., Tokyo 108 (JP)
(72) Erfinder: ANDREE, Roland, D-40764 Langenfeld (DE); DREWES, Mark, Wilhelm, D-40764 Langenfeld (DE); SCHALLNER, Otto, D-40789 Monheim (DE); DOLLINGER, Markus, D-51381 Leverkusen (DE); SANTEL, Hans-Joachim, D-51371 Leverkusen (DE); ERDELEN, Christoph, D-42799 Leichlingen (DE); YANAGI, Akihiko, Tochigi (JP); GOTO, Toshio, Kokubunji-machi Tochigi (JP)
(74) Vertreter: Linkenheil, Dieter
(86) Internationale Anmeldenummer: EP9602603
(87) Internationale Veröffentlichungsnummer: WO97001541

(56) Entgegenhaltungen:
- EP-A- 0 255 047
- EP-A- 0 260 621
- EP-A- 0 408 382
- WO-A-95/32952
- WO-A-96/24590

## Beschreibung

Die Erfindung betrifft neue substituierte Cyanophenyluracile, Verfahren zu ihrer Herstellung sowie ihre Verwendung als Pflanzenbehandlungsmittel, insbesondere als Herbizide und Insektizide.

Es ist bekannt, daß bestimmte substituierte Phenyluracile herbizide Eigenschaften aufweisen (vgl. EP-408 382/US-5 084 084/US-5 127 935/US-5 154 755, EP-563 384, EP-648 749, WO 91/00278, US-4 979 982, US-5 169 430). Diese Verbindungen haben jedoch bisher keine nennenswerte Bedeutung erlangt.

Es wurden nun die neuen substituierten Cyanophenyluracile der allgemeinen Formel (I) gefunden 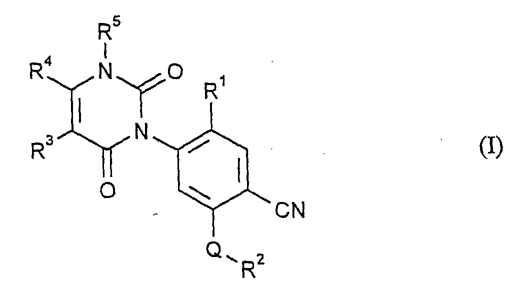 in welcher
- Q: für O, S, SO oder SO₂ steht,
- R¹: für Wasserstoff, Cyano, Fluor oder Chlor steht,
- R²: für jeweils gegebenenfalls durch Cyano, Carboxy, Nitro, Carbamoyl, Thiocarbamoyl, Fluor, Chlor, Brom, durch C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Alkyl-carbonyl oder C₁-C₄-Alkoxyl-carbonyl (welche jeweils gegebenenfalls durch Fluor und/oder Chlor substituiert sind), durch Phenyl, Phenoxy oder Phenylthio (welche jeweils gegebenfalls durch Fluor, Chlor, Brom, Cyano, Methyl, Methoxy, Trifluormethyl und/oder Trifluormethoxy substituiert sind) substituiertes Aryl mit 6 oder 10 Kohlenstoffatomen steht,
- R³: für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
- R⁴: für gegebenenfalls durch Fluor und/oder Chlor substituiertes Alkyl mit 1 bis 4 Kohlenstoffatomen steht und
- R⁵: für Wasserstoff oder für jeweils gegebenenfalls durch Fluor, Chlor oder C₁-C₄-Alkoxy substituiertes Alkyl, Alkoxy, Alkenyl oder Alkinyl mit jeweils bis zu 6 Kohlenstoffatomen steht.

Man erhält die neuen substituierten Cyanophenyluracile der allgemeinen Formel (I), wenn man
(a) substituierte Halogenphenyluracile der allgemeinen Formel (II) 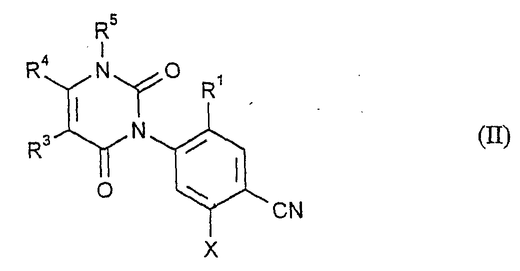 in welcher
   R¹, R³, R⁴ und R⁵ die oben angegebenen Bedeutungen haben und
   - X: für Halogen steht,
   mit nucleophilen Verbindungen der allgemeinen Formel (III)

   M-Q-R² (III)

   in welcher
   Q und R² die oben angegebenen Bedeutungen haben und
   - M: für Wasserstoff oder ein Alkalimetall steht,
   gegebenenfalls in Gegenwart eines Reaktionshilfsmittels und gegebenenfalls in Gegenwart eines Verdünnungsmittels umsetzt,
   oder wenn man
(b) substituierte Cyanophenyluracile der allgemeinen Formel (Ia) 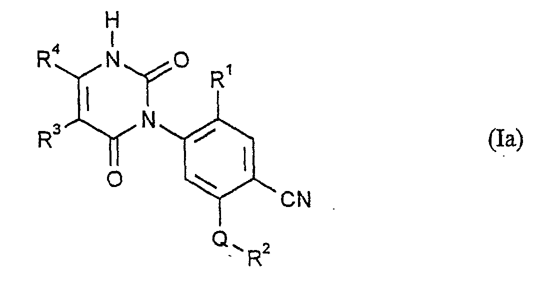 in welcher
   Q, R¹, R², R³ und R⁴ die oben angegebenen Bedeutungen haben,
   mit einem Alkylierungsmittel gegebenenfalls in Gegenwart eines Reaktionshilfsmittels und gegebenenfalls in Gegenwart eines Verdünnungsmittels umsetzt.

Die neuen substituierten Cyanophenyluracile der allgemeinen Formel (I) zeichnen sich durch starke herbizide und insektizide Wirksamkeit aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I),
in welcher
- Q: für O, S, SO oder SO₂ steht,
- R¹: für Wasserstoff, Fluor oder Chlor steht,
- R²: für jeweils gegebenenfalls durch Cyano, Carboxy, Nitro, Carbamoyl, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl substituiertes Phenyl steht, oder
- R³: für Wasserstoff, Fluor, Chlor, Brom oder Methyl steht,
- R⁴: für Methyl, Ethyl, Difluormethyl, Dichlormethyl, Trifluormethyl, Trichlormethyl, Chlordifluormethyl, Fluordichlormethyl, Chlorethyl, Fluorethyl, Dichlorethyl, Dichlorethyl, Chlorfluorethyl, Chlordifluorethyl, Fluordichlorethyl, Trifluorethyl, Tetrafluorethyl, Chlortrifluorethyl oder Pentafluorethyl steht und
- R⁵: für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i- oder s-Butoxy, Propenyl, Butenyl, Propinyl oder Butinyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

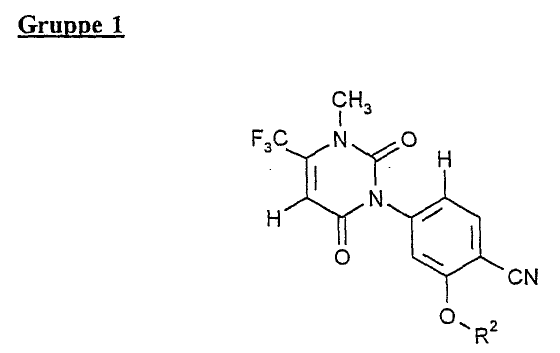

R² hat hierbei beispielhaft die in der nachstehenden Aufzählung angegebenen Bedeutungen: Phenyl, Cyanophenyl, Carboxyphenyl, Nitrophenyl, Fluorphenyl, Chlorphenyl, Bromphenyl, Methylphenyl, Trifluormethylphenyl, Methoxyphenyl, Difluormethoxyphenyl, Trifluormethoxyphenyl, Methoxycarbonylphenyl, Ethoxycarbonylphenyl,

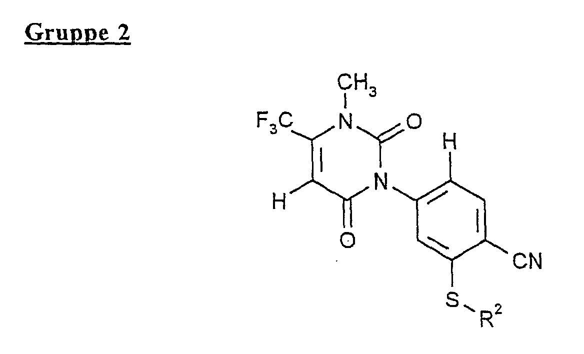

R² hat hierbei beispielhaft die oben in Gruppe 1 aufgeführten Bedeutungen.

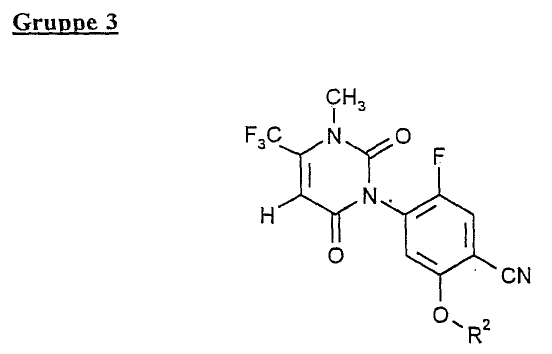

R² hat hierbei beispielhaft die oben in Gruppe 1 aufgeführten Bedeutungen.

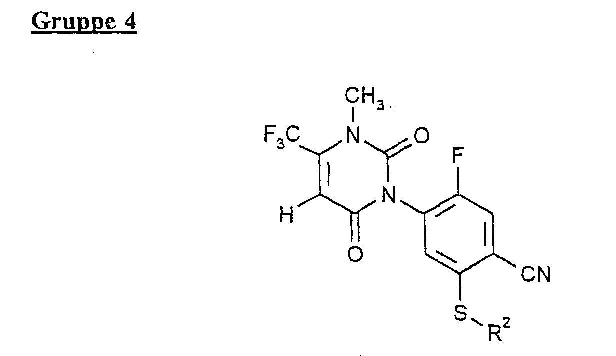

R² hat hierbei beispielhaft die oben in Gruppe 1 aufgeführten Bedeutungen.

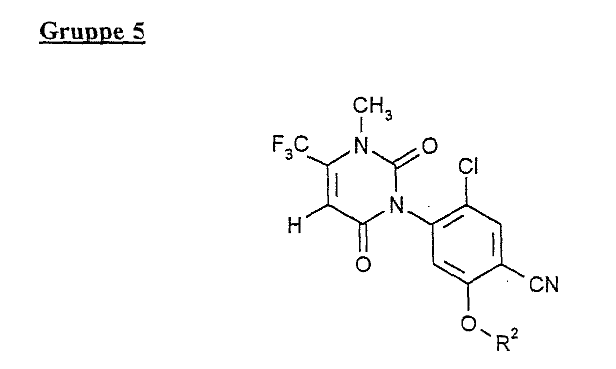

R² hat hierbei beispielhaft die oben in Gruppe 1 aufgeführten Bedeutungen.

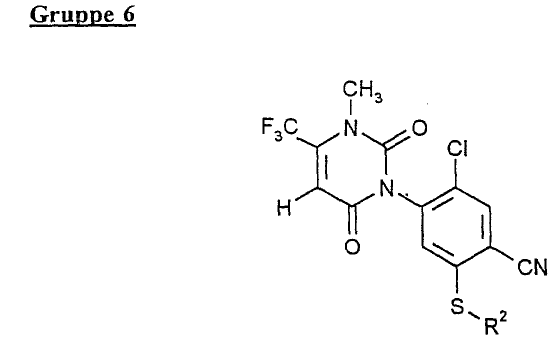

R² hat hierbei beispielhaft die oben in Gruppe 1 aufgeführten Bedeutungen.

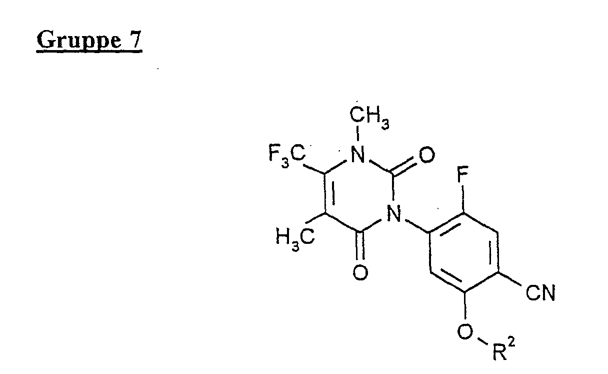

R² hat hierbei beispielhaft die oben in Gruppe 1 aufgeführten Bedeutungen.

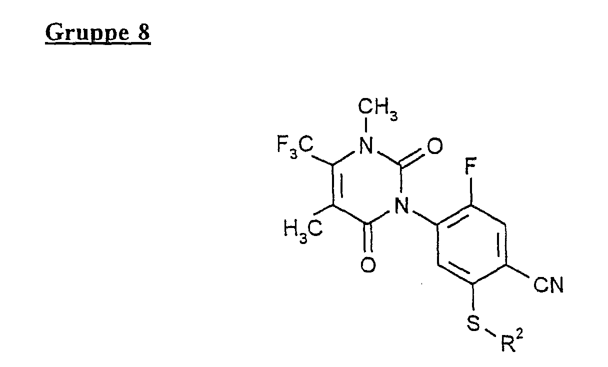

R² hat hierbei beispielhaft die oben in Gruppe 1 aufgeführten Bedeutungen.

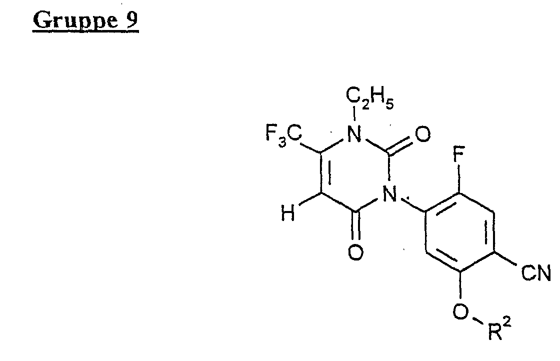

R² hat hierbei beispielhaft die oben in Gruppe 1 aufgeführten Bedeutungen.

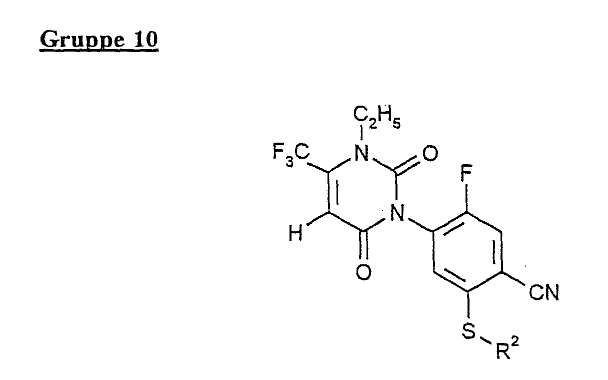

R² hat hierbei beispielhaft die oben in Gruppe 1 aufgeführten Bedeutungen.

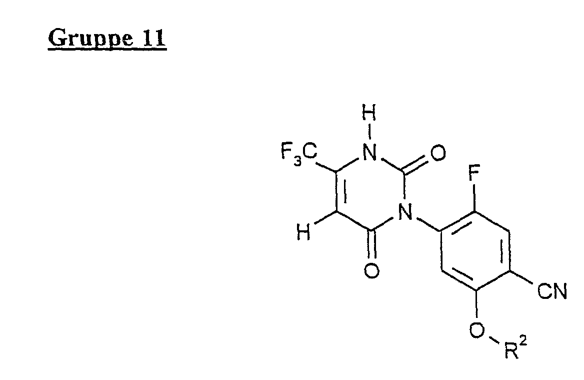

R² hat hierbei beispielhaft die oben in Gruppe 1 aufgeführten Bedeutungen.

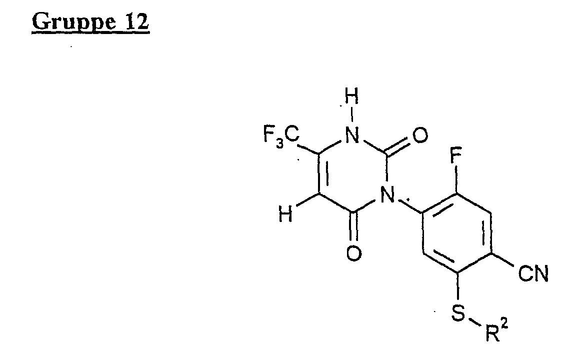

R² hat hierbei beispielhaft die oben in Gruppe 1 aufgeführten Bedeutungen.

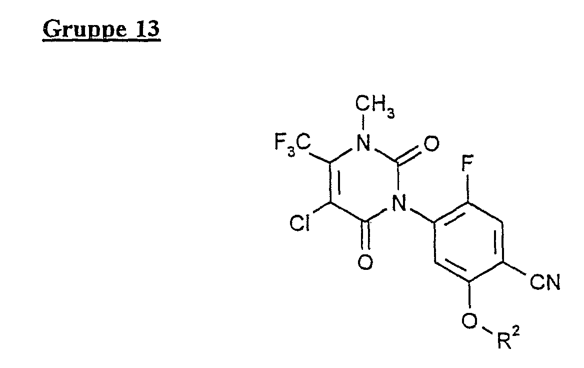

R² hat hierbei beispielhaft die oben in Gruppe 1 aufgeführten Bedeutungen.

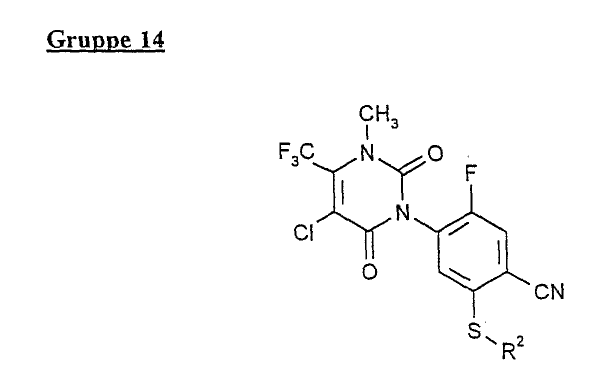

R² hat hierbei beispielhaft die oben in Gruppe 1 aufgeführten Bedeutungen.

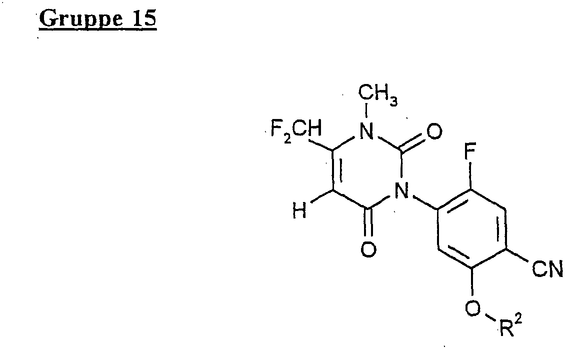

R² hat hierbei beispielhaft die oben in Gruppe 1 aufgeführten Bedeutungen.

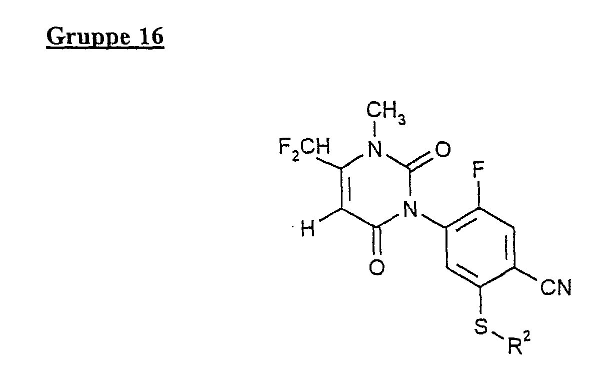

R² hat hierbei beispielhaft die oben in Gruppe 1 aufgeführten Bedeutungen.

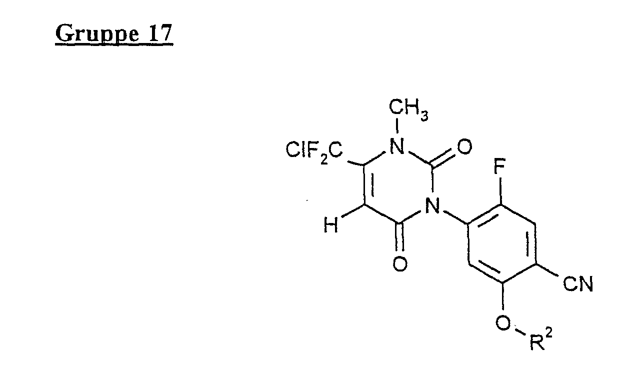

R² hat hierbei beispielhaft die oben in Gruppe 1 aufgeführten Bedeutungen.

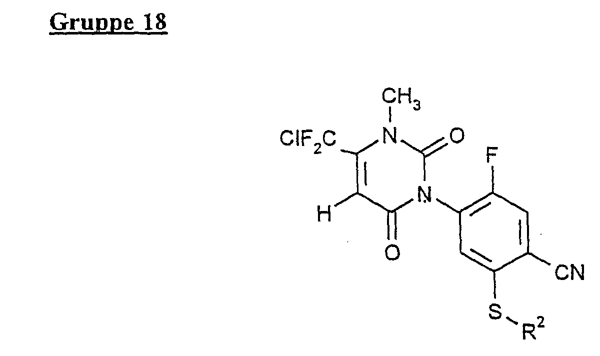

R² hat hierbei beispielhaft die oben in Gruppe 1 aufgeführten Bedeutungen.

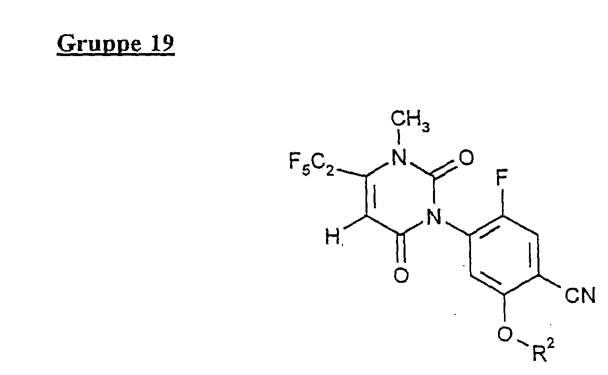

R² hat hierbei beispielhaft die oben in Gruppe 1 aufgeführten Bedeutungen.

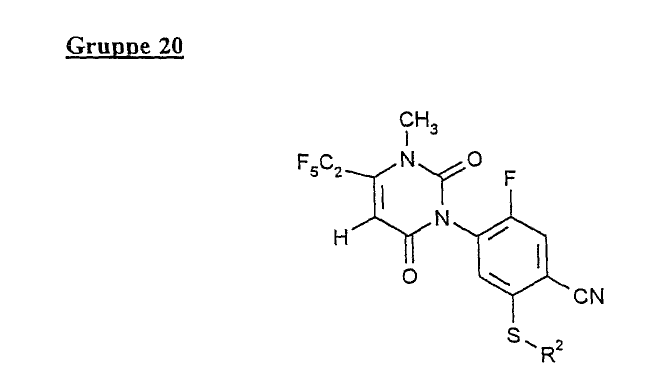

R² hat hierbei beispielhaft die oben in Gruppe 1 aufgeführten Bedeutungen.

Die beim erfindungsgemäßen Verfahren zur Herstellung der Verbindungen der Formel (I) als Ausgangsstoffe zu verwendenden substituierten Halogenphenyluracile sind durch die Formel (II) allgemein definiert. In der Formel (II) haben R¹, R³, R⁴ und R⁵ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R¹, R³, R⁴ und R⁵ angegeben wurde; X steht vorzugsweise für Fluor, Chlor oder Brom, insbesondere für Fluor.

Die Ausgangsstoffe der Formel (II) sind bekannt und/oder können nach an sich bekannten Verfahren hergestellt werden (vgl. EP 648749, Herstellungsbeispiele).

Die beim erfindungsgemäßen Verfahren zur Herstellung der Verbindungen der Formel (I) weiter als Ausgangsstoffe zu verwendenden nucleophilen Verbindungen sind durch die Formel (III) allgemein definiert. In der Formel (III) haben Q und R² vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für Q und R² angegeben wurden; M steht vorzugsweise für Wasserstoff, Lithium, Natrium oder Kalium, insbesondere für Wasserstoff, Natrium oder Kalium.

Die Ausgangsstoffe der Formel (III) sind bekannte Synthesechemikalien.

Das erfindungsgemäße Verfahren (a) zur Herstellung der Verbindungen der Formel (I) wird vorzugsweise in Gegenwart eines geeigneten Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calciumamid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8 Diazabicyclo[5,4,0]-undec-7-en (DBU).

Das erfindungsgemäße Verfahren (a) zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethylt-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -ipropylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder -monoethylether, Diethylenglycol-monomethylether oder -monoethylether; deren Gemische mit Wasser oder reines Wasser.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 200°C, vorzugsweise zwischen 10°C und 150°C.

Das erfindungsgemäße Verfahren (a) wird im allgemeinen unter Normaldruck durchgeführt. Es ist jedoch auch möglich, das erfzndungsgemäße Verfahren unter erhöhtem oder vermindertem Druck - im allgemeinen zwischen 0,1 bar und 10 bar - durchzuführen.

Zur Durchführung des erfindungsgemäßen Verfahrens (a) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die beim erfindungsgemäßen Verfahren (b) zur Herstellung der Verbindungen der Formel (I) als Ausgangsstoffe zu verwendenden substituierten Cyanophenyluracile sind durch die Formel (Ia) allgemein definiert. In der Formel (Ia) haben Q, R¹, R², R³ und R⁴ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für Q, R¹, R², R³ und R⁴ angegeben wurde.

Die Ausgangsstoffe der Formel (Ia) sind erfindungsgemäße, neue Verbindungen; sie können nach dem erfindungsgemäßen Verfahren (a) hergestellt werden.

Das erfindungsgemäße Verfahren (b) wird unter Verwendung eines Alkylierungsmittels durchgeführt. Unter Alkylierungsmitteln sind in diesem Zusammenhang vorzugsweise gegebenenfalls durch Fluor, Chlor oder C₁-C₄-Alkoxy substituierte Alkyl-, Alkenyl- oder Alkinylhalogenide (insbesondere Chloride, Bromide oder Iodide) mit jeweils bis zu 6 Kohlenstoffatomen oder Dialkylsulfate mit bis zu 6 Kohlenstoffatomen in den Alkylgruppen zu verstehen. Es handelt sich hierbei um bekannte Synthesechemikalien.

Das erfindungsgemäße Verfahren (b) zur Herstellung der Verbindungen der Formel (I) wird vorzugsweise in Gegenwart eines geeigneten Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kaliummethanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo-[2,2,2]-octan (DABCO), 1,5-Diazabicyclo[4,3,0]-non-5-en (DBN), und 1,8 Diazabicyclo[5,4,0]-undec-7-en (DBU).

Das erfindungsgemäße Verfahren (b) zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethylt-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -ipropylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder -monoethylether, Diethylenglycol-monomethylether oder -monoethylether; deren Gemische mit Wasser oder reines Wasser.

Die Reaktionstemperaturen können bei der Durchrührung des erfindungsgemäßen Verfahrens (b) in einem größeren Bereich variiert werden. Im allgemeinene arbeitet man bei Temperaturen zwischen 0°C und 150°C, vorzugsweise zwischen 10°C und 120°C.

Das erfindungsgemäße Verfahren (b) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im NachauflaufVerfahren.

Zu Anwendung geeignete Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,

Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,

Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,

Edifenphos, Epoxyconazole, Ethirimol, Etridiazol,

Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

Guazatine,

Hexachlorobenzol, Hexaconazol, Hymexazol,

Imazalil, Imibenconazol, Iminoctadin, Iprobenfos (IBP), Iprodion, Isoprothiolan,

Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Mepanipyrim, Mepronil, Metalaxyl, Metconazol, Methasulfocarb, Methfuroxam, Metiram, Metsulfovax, Myclobutanil,

Nickel-dimethyldithiocarbamat, Nitrothal-isopropyl, Nuarimol,

Ofurace, Oxadixyl, Oxamocarb, Oxycarboxin,

Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,

Quintozen (PCNB),

Schwefel und Schwefel-Zubereitungen,

Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,

Validamycin A, Vinclozolin,

Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,

Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,

Edifenphos, Emamectin, Esfenvalerat, Ethiofencarb, Ethion, Ethofenprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,

Imidacloprid, Iprobenfos, Isazophos, Isofenphos, Isoprocarb, Isoxathion, Ivermectin, Lambda-cyhalothrin, Lufenuron,

Malathion, Mecarbam, Mervinphos, Mesulfenphos, Metaldehyd,. Methacrifos, Methamidophos, Methidathion, Methiocarb, Methomyl, Metolcarb, Milbemectin, Monocrotophos, Moxidectin,

Naled, NC 184, NI 25, Nitenpyram,

Omethoat, Oxamyl, Oxydemethon M, Oxydeprofos,

Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,

Quinalphos,

RH 5992,

Salithion, Sebufos, Silafluofen, Sulfotep, Sulprofos,

Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,

Vamidothion, XMC, Xylylcarb, YI 5301/5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgierund/oder schaumerzeugende Mittel kommen in Frage: z.B. nicht-ionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide in Frage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifpp-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluarochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

## Patentansprüche

1. Cyanophenyluracile der allgemeinen Formel (I) 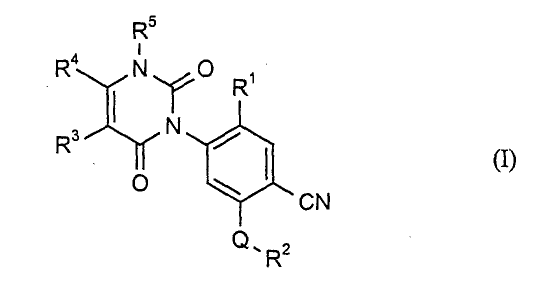 in welcher
Q für O, S, SO oder SO₂ steht,
R¹ für Wasserstoff, Cyano, Fluor oder Chlor steht,
R² für jeweils gegebenenfalls durch Cyano, Carboxy, Nitro, Carbamoyl, Thiocarbamoyl, Fluor, Chlor, Brom, durch C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Alkyl-carbonyl oder C₁-C₄-Alkoxy-carbonyl (welches jeweils gegebenenfalls durch Fluor und/ oder Chlor sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl mit 6 oder 10 Kohlenstoffatomen im Arylteil steht,
R³ für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
R⁴ für gegebenenfalls durch Fluor und/oder Chlor substituiertes Alkyl mit 1 bis 4 Kohlenstoffatomen steht und
R⁵ für Wasserstoff oder für jeweils gegebenenfalls durch Fluor, Chlor oder C₁-C₄-Alkoxy substituiertes Alkyl, Alkoxy, Alkenyl oder Alkinyl mit jeweils bis zu 6 Kohlenstoffatomen steht.

2. Cyanophenyluracile der allgemeinen Formel (I) gemäß Anspruch 1, **dadurch gekennzeichnet, daß**
Q für O, S, SO oder SO₂ steht,
R¹ für Wasserstoff, Fluor oder Chlor steht,
R² für jeweils gegebenenfalls durch Cyano, Carboxy, Nitro, Carbamoyl, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl oder n- oder i-Propoxycarbonyl substituiertes Phenyl steht, oder
R² weiterhin für jeweils gegebenenfalls durch Cyano, Carboxy, Carbamoyl, Thiocarbamoyl, Fluor, Chlor, Brom, Methyl, Ethyl, n- oder i-Propyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl, durch Phenyl, Phenoxy oder Phenylthio substituiertes substituiertes Furyl, Tetrahydrofuryl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,
R³ für Wasserstoff, Fluor, Chlor, Brom oder Methyl steht,
R⁴ für Methyl, Ethyl, Difluormethyl, Dichlormethyl, Trifluormethyl, Trichlormethyl, Chlordifluormethyl, Fluordichlormethyl, Chlorethyl, Fluorethyl, Dichlorethyl, Dichlorethyl, Chlorfluorethyl, Chlordifluorethyl, Fluordichlorethyl, Trifluorethyl, Tetrafluorethyl, Chlortrifluorethyl oder Pentafluorethyl steht und
R⁵ für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i- oder s-Butoxy, Propenyl, Butenyl, Propinyl oder Butinyl steht.

3. Cyanophenyluracile der allgemeinen Formel (Ia) 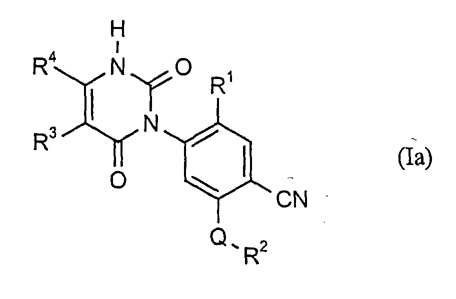 **dadurch gekennzeichnet, daß**
Q, R¹, R², R³ und R⁴ die in Anspruch 1 angegebenen Bedeutungen haben.

4. Verfahren zur Herstellung von Cyanophenyluracilen der allgemeinen Formel (I) 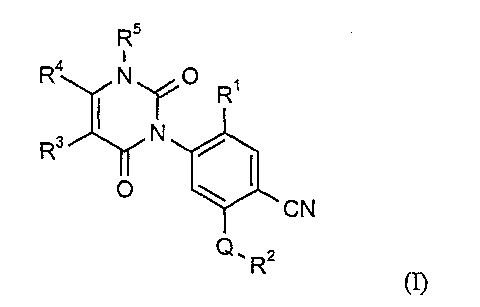 in welcher
R¹, R², R³, R⁴, R⁵ und Q die in Anspruch 1 genannten Bedeutungen haben,
**dadurch gekennzeichnet, daß** man
(a) substituierte Halogenphenyluracile der allgemeinen Formel (II) 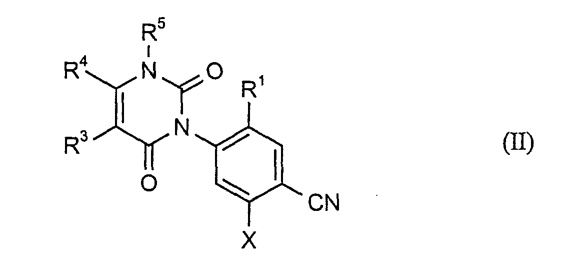 in welcher
R¹, R³, R⁴ und R⁵ die oben angegebenen Bedeutungen haben und
X für Halogen steht,
mit nucleophilen Verbindungen der allgemeinen Formel (III)
M-Q-R² (III)
in welcher
Q und R² die oben angegebenen Bedeutungen haben und
M für Wasserstoff oder ein Alkalimetall steht,
gegebenenfalls in Gegenwart eines Reaktionshilfsmittels und gegebenenfalls in Gegenwart eines Verdünnungsmittels umsetzt,
oder daß man
(b) substituierte Cyanophenyluracile der allgemeinen Formel (Ia) 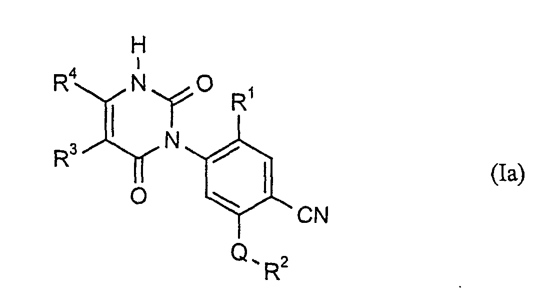
in welcher
Q, R¹, R², R³ und R⁴ die oben angegebenen Bedeutungen haben,
mit einem Alkylienmgsmittel gegebenenfalls in Gegenwart eines Reaktionshilfsmittels und gegebenenfalls in Gegenwart eines Verdünnungsmittels umsetzt.

5. Herbizide Mittel, **gekennzeichnet durch** einen Gehalt an mindestens einem Cyanophenyluracil der allgemeinen Formel (I) gemäß einem der Ansprüche 1 bis 3.

6. Verfahren zur Bekämpfung von unerwünschten Pflanzen, **dadurch gekennzeichnet, daß** man Cyanophenyluracile der allgemeinen Formel (I) gemäß einem der Ansprüche 1 bis 3 auf unerwünschte Pflanzen und/oder ihre Lebensräume einwirken läßt.

7. Verwendung von Cyanophenyluracilen der allgemeinen Formel (I) gemäß einem der Ansprüche 1 bis 3 zur Bekämpfung von unerwünschten Pflanzen.

8. Verfahren zur Herstellung von herbiziden Mitteln, **dadurch gekennzeichnet, daß** man Cyanophenyluracile der allgemeinen Formel (I) gemäß einem der Ansprüche 1 bis 3 mit Streckmitteln und/oder oberflächenaktiven Substanzen vermischt.

## Claims

1. Cyanophenyluracils of the general formula (I) 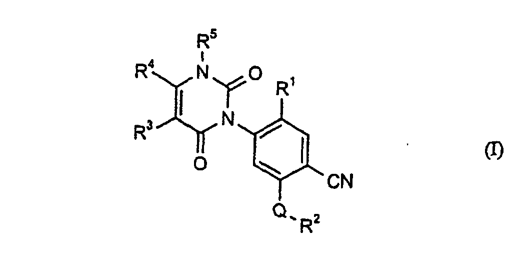 in which
Q represents O, S, SO or SO₂,
R¹ represents hydrogen, cyano, fluorine or chlorine,
R² represents respectively optionally cyano-, carboxyl-, nitro-, carbamoyl-, thiocarbomyl-, fluorine-, chlorine-, bromine-, or C₁-C₄-alkyl-, C₁-C₄-alkoxy-, C₁-C₄-alkylthio-, C₁-C₄-alkylsulphinyl-, C₁-C₄-alkylsulphonyl-, C₁-C₄-alkyl-carbonyl- or C₁-C₄-alkoxy-carbonyl- (each of which is optionally by fluorine and/or chlorine), or phenyl-, phenoxy- or phenylthio- (each of which is optionally substituted by fluorine, chlorine, bromine, cyano, methyl, methoxy, trifluoromethyl and/or trifluoromethoxy) substituted aryl having 6 or 10 carbon atoms in the aryl moiety,
R³ represents hydrogen, fluorine, chlorine, bromine or represents respectively optionally fluorine- and/or chlorine-substituted alkyl or alkoxy having in each case 1 to 4 carbon atoms,
R⁴ represents optionally fluorine- and/or chlorine-substituted alkyl having 1 to 4 carbon atoms and
R⁵ represents hydrogen or represents respectively optionally fluorine-, chlorine- or C₁-C₄-alkoxy-substituted alkyl, alkoxy, alkenyl or alkinyl having in each case up to 6 carbon atoms.

2. Cyanophenyluracils of the general formula (I) according to Claim 1, **characterized in that**
Q represents O, S, SO or SO₂ ,
R¹ represents hydrogen, fluorine or chlorine,
R² represents respectively optionally cyano-, carboxyl-, nitro-, carbamoyl-, thiocarbamoyl-, fluorine-, chlorine-, bromine-, methyl-, ethyl-, n- or i-propyl-, n-, i-, s- or t-butyl-, trifluoro-methyl-, methoxy-, ethoxy-, n- or i-propoxy-, methylthio-, ethylthio-, methylsulphinyl-, ethylsulphinyl-, methylsulphonyl-, ethylsulphonyl-, acetyl-, propionyl-, methoxycarbonyl-, ethoxycarbonyl- or n- or i-propoxycarbonyl-substituted phenyl, or
R² furthermore represents respectively optionally cyano-, carboxyl-, carbamoyl-, thiocarbamoyl-, fluorine-, chlorine-, bromine-, methyl-, ethyl-, n- or i-propyl-, methoxy-, ethoxy-, n- or i-propoxy-, methylthio-, ethylthio-, methylsulphinyl-, ethylsulphinyl-, methylsulphonyl-, ethylsulphonyl-, acetyl-, propionyl-, methoxycarbonyl-, ethoxycarbonyl-, n- or i-propoxycarbonyl-, or phenyl-, phenoxy- or phenylthio-substituted substituted furyl, tetrahydrofuryl, thienyl, tetrahydrothienyl, oxetanyl, thietanyl, oxazolyl, isoxazolyl, thiazolyl, oxadiazolyl, thiadiazolyl, pyrazolyl, pyridinyl, pyrimidinyl, triazinyl, indolyl, quinolinyl or quinoxalinyl,
R³ represents hydrogen, fluorine, chlorine, bromine or methyl,
R⁴ represents methyl, ethyl, difluoromethyl, dichloromethyl, trifluoromethyl, trichloromethyl, chlorodifluoromethyl, fluorodichloromethyl, chloroethyl, fluoroethyl, dichloroethyl, dichloroethyl, chlorofluoroethyl, chlorodifluoroethyl, fluorodichloroethyl, trifluoroethyl, tetrafluoroethyl, chlorotrifluoroethyl or pentafluoroethyl and
R⁵ represents hydrogen or represents respectively optionally fluorine-, chlorine-, methoxy- or ethoxy-substituted methyl, ethyl, n- or i- propyl, n-, i-, s- or t-butyl, methoxy, ethoxy, n- or i-propoxy, n-, i- or s-butoxy, propenyl, butenyl, propinyl or butinyl.

3. Cyanophenyluracils of the general formula (1a) 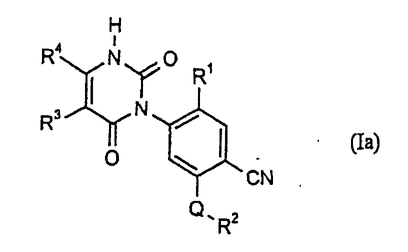 **characterized in that**
Q, R¹, R², R³ and R⁴ are each as defined in Claim 1.

4. Process for preparing cyanophenyluracils of the general formula (I) 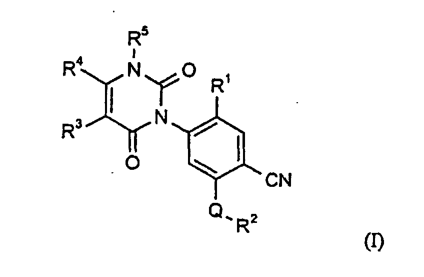 in which
R¹, R², R³, R⁴, R⁵ and Q are each as defined in Claim 1,
**characterized in that**
(a) substituted halogenophenyluracils of the general formula (II) 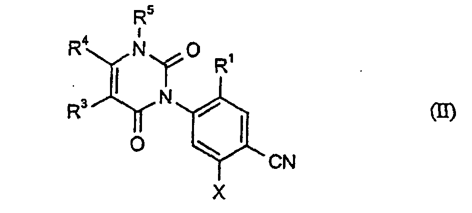 in which
R¹, R³, R⁴ and R⁵ are each as defined above and
X represents halogen,
are reacted with nucleophilic compounds of the general formula (III)
M-Q-R² (III)
in which
Q and R² are each as defined above and
M represents hydrogen or an alkali metal,
if appropriate in the presence of a reaction auxiliary and if appropriate in the presence of a diluent,
or
(b) substituted cyanophenyluracils of the general formula (Ia) 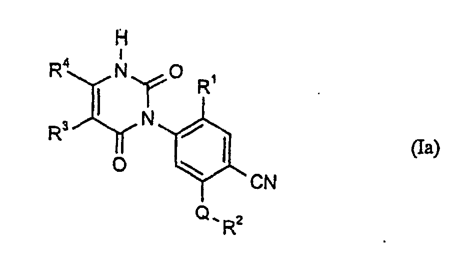 in which
Q, R¹, R², R³ and R⁴ are each as defined above
are reacted with an alkylating agent, if appropriate in the presence of a reaction auxiliary and if appropriate in the presence of a diluent.

5. Herbicides, **characterized by** a content of at least one cyanophenyluracil of the general formula (I) according to one of Claims 1 to 3.

6. Method for controlling undesirable plants, **characterized in that** cyanophenyluracils of the general formula (I) according to one of Claims 1 to 3 are allowed to act on undesirable plants and/or their habitats.

7. Use of cyanophenyluracils of the general formula (I) according to one of Claims 1 to 3 for controlling undesirable plants.

8. Process for preparing herbicides, **characterized in that** cyanophenyluracils of the general formula (I) according to one of Claims 1 to 3 are mixed with extenders and/or surfactants.

## Revendications

1. Cyanophényluraciles de formule générale (I) 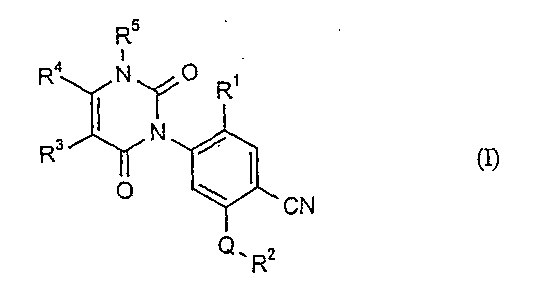 dans laquelle
Q représente O, S, SO ou SO₂,
R¹ est l'hydrogène, un groupe cyano, le fluor ou le chlore,
R² représente un groupe aryle ayant 6 ou 10 atomes de carbone dans la partie aryle, éventuellement substitué dans chaque cas par un reste cyano, carboxy, nitro, carbamoyle, thiocarbamoyle, du fluor, du chlore, du brome, par un reste alkyle en C₁ à C₄, alkoxy en C₁ à C₄, alkylthio en C₁ à C₄, alkylsulfinyle en C₁ à C₄, alkylsulfonyle en C₁ à C₄ (alkyle en C₁ à C₄)carbonyle ou (alkoxy en C₁ à C₄)carbonyle (chacun étant éventuellement substitué par du fluor et/ou du chlore), par un reste phényle, phénoxy ou phénylthio (chacun étant éventuellement substitué par du fluor, du chlore, du brome, un radical cyano, méthyle, méthoxy, trifluorométhyle et/ou trifluorométhoxy),
R³ représente l'hydrogène, le fluor, le chlore, le brome ou un groupe alkyle ou alkoxy ayant chacun 1 à 4 atomes de carbone, chacun étant éventuellement substitué par du fluor et/ou du chlore,
R⁴ est un groupe alkyle ayant 1 à 4 atomes de carbone éventuellement substitué par du fluor et/ou du chlore, et
R⁵ représente l'hydrogène ou un groupe alkyle, alkoxy, alcényle ou alcynyle ayant chacun jusqu'à 6 atomes de carbone et étant chacun éventuellement substitué par du fluor, du chlore ou un radical alkoxy en C₁ à C₄.

2. Cyanophényluraciles de formule générale (I) suivant la revendication 1, **caractérisés en ce que**
Q représente O, S, SO ou SO₂,
R¹ est l'hydrogène, le fluor ou le chlore,
R² représente un groupe phényle éventuellement substitué par un radical cyano, carboxy, nitro, carbamoyle, thiocarbamoyle, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, méthylthio, éthylthio, méthylsulfinyle, éthylsulfinyle, méthylsulfonyle, éthylsulfonyle, acétyle, propionyle, méthoxycarbonyle, éthoxycarbonyle, n-propoxycarbonyle ou isopropoxycarbonyle, ou bien
R² représente en outre un reste furyle, tétrahydrofuryle, thiényle, tétrahydrothiényle, oxétannyle, thiétannyle, oxazolyle, isoxazolyle, thiazolyle, oxadiazolyle, thiadiazolyle, pyrazolyle, pyridinyle, pyrimidinyle, triazinyle, indolyle, quinolinyle ou quinoxalinyle dont chacun est éventuellement substitué par un radical cyano, carboxy, carbamoyle, thiocarbamoyle, fluoro, chloro, bromo, méthyle, éthyle, n-propyle, isopropyle, méthoxy, éthoxy, n-propoxy, isopropoxy, méthylthio, éthylthio, méthylsulfinyle, éthylsulfinyle, méthylsulfonyle, éthylsulfonyle, acétyle, propionyle, méthoxycarbonyle, éthoxycarbonyle, n-propoxycarbonyle, isopropoxycarbonyle, par un radical phényle, phénoxy ou phénylthio,
R³ représente l'hydrogène, le fluor, le chlore, le brome ou le radical méthyle,
R⁴ est un reste méthyle, éthyle, difluorométhyle, dichlorométhyle, trifluorométhyle, trichlorométhyle, chlorodifluorométhyle, fluorodichlorométhyle, chloréthyle, fluoréthyle, dichloréthyle, dichloréthyle, chlorofluoréthyle, chlorodifluoréthyle, fluorodichloréthyle, trifluoréthyle, tétrafluoréthyle, chlorotrifluoréthyle ou pentafluoréthyle et
R⁵ représente l'hydrogène ou un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, propényle, butényle, propynyle ou butynyle éventuellement substitué par du fluor, du chlore, un radical méthoxy ou éthoxy.

3. Cyanophényluraciles de formule générale (Ia) 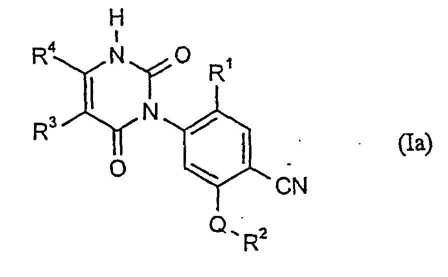 **caractérisés en ce que** Q, R¹, R², R³ et R⁴ ont les définitions indiquées dans la revendication 1.

4. Procédé de production de cyanophényluraciles de formule générale (I) 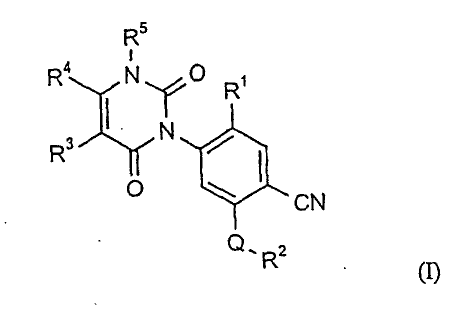 dans laquelle
R¹, R², R³, R⁴, R⁵ et Q ont les définitions indiquées dans la revendication 1,
**caractérisé en ce que** :
(a) on fait réagir des halogénophényluraciles substitués de formule générale (II) 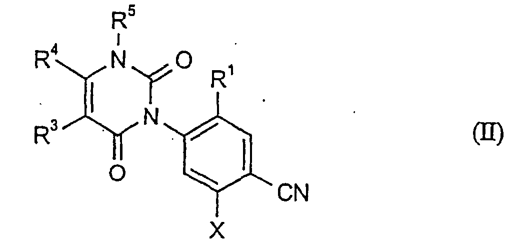 dans laquelle
R¹, R³, R⁴ et R⁵ ont les définitions indiquées ci-dessus et
X représente un halogène,
avec des composés nucléophiles de formule générale (III)
M-Q-R² (III)
dans laquelle
Q et R² ont les définitions indiquées ci-dessus et
M représente l'hydrogène ou un métal alcalin,
le cas échéant en présence d'un auxiliaire de réaction et en présence éventuelle d'un diluant,
ou **en ce que** :
(b) on fait réagir des cyanophényluraciles substitués de formule générale (Ia) 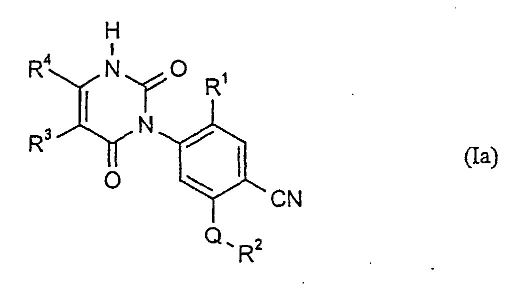
dans laquelle
Q, R¹, R², R³ et R⁴ ont les définitions données ci-dessus,
avec un agent d'alkylation, éventuellement en présence d'un auxiliaire de réaction et, le cas échéant en présence d'un diluant.

5. Compositions herbicides, **caractérisées par** une teneur en au moins un cyanophényluracile de formule générale (I) suivant l'une des revendications 1 à 3.

6. Procédé pour combattre des plantes indésirables, **caractérisé en ce qu'**on fait agir des cyanophényluraciles de formule générale (I) suivant l'une des revendications 1 à 3 sur les plantes indésirables et/ou sur leur milieu.

7. Utilisation de cyanophényluraciles de formule générale (I) suivant l'une des revendications 1 à 3 pour combattre des plantes indésirables.

8. Procédé de préparation de compositions herbicides, **caractérisé en ce qu'**on mélange des cyanophényluraciles de formule générale (I) suivant l'une des revendications 1 à 3 avec des diluants et/ou des substances tensio-actives.
